# FASCICULE DE BREVET EUROPEEN

(11) **EP 3 090 082 B1**
(45) Date de publication et mention de la délivrance du brevet: **15.11.2017**
(21) Numéro de dépôt: 15702513.1
(22) Date de dépôt: 05.01.2015
(51) Int. Cl.: A41D 13/12, A61B 5/00, D03D 1/00, D03D 11/02, D03D 15/08, A41D 1/00

(54) **VÊTEMENT INSTRUMENTE COMPORTANT UN TEXTILE TISSE ELASTIQUE**
INSTRUMENTIERTES KLEIDUNGSSTÜCK MIT EINEM ELASTISCHEN GEWEBE
INSTRUMENTED GARMENT COMPRISING AN ELASTIC WOVEN FABRIC

(30) Priorité: 03.01.2014 FR 1450035
(43) Date de publication de la demande: 09.11.2016
(73) Titulaire: City Zen Sciences, 75011 Paris (FR); Payen, 07000 Saint Julien en Saint Alban (FR)
(72) Inventeur: FICHET, Laurent, F-01340 Villars les Dombes (FR); BIENSTMAN, William, F-69003 Lyon (FR); DIEPPOIS, Didier, F-26000 Valence (FR)
(74) Mandataire: Cabinet Laurent & Charras
(86) Numéro de dépôt international: PCT/FR2015/050002
(87) Numéro de publication internationale: WO 2015/101759

(56) Documents cités:
- WO-A2-99/15722
- GB-A- 1 413 024
- US-A1- 2003 211 797
- US-A1- 2005 054 941
- US-A1- 2006 111 640
- US-A1- 2006 124 193
- US-A1- 2006 228 970
- US-A1- 2011 073 353

## Description

### Domaine technique

L'invention se rapporte au domaine des vêtements instrumentés, notamment de sport ou pour le suivi médical d'un patient. Ces vêtements sont le plus souvent élastiques et équipés de capteurs. De tels capteurs sont généralement associés à un textile de manière à permettre de réaliser une mesure d'une caractéristique physiologique et/ou actimétrique du porteur du vêtement.

L'invention vise donc plus particulièrement les vêtements dit « intelligents » connectés entre eux et/ou à un organe annexe informatique permettant par exemple de visualiser une représentation graphique fonction de la mesure effectuée par le capteur. Par ailleurs, les capteurs utilisés pour ce type d'application peuvent se présenter sous différentes formes et notamment : les capteurs permettant de mesurer la fréquence et la variabilité cardiaque (électrodes ECG), la fréquence respiratoire, la température cutanée, le flux thermique, le flux hydrique, l'oxygénation sanguine, l'oxygénation musculaire, la composition chimique de la sueur, la bio-impédance, la position, la vitesse ou l'accélération de tout ou partie du corps du porteur du vêtement.

### Techniques antérieures

De façon générale, les vêtements instrumentés dans lesquels des capteurs sont agencés, présentent des fils électriques conducteurs qui peuvent être tissés directement avec les fils de chaîne et de trame à l'intérieur du tissu. Un tel mode de réalisation est notamment décrit dans le document WO 2005/032447 dans lequel des fils élastiques conducteurs peuvent être simplement tissés avec des fils élastiques non conducteurs.

Cependant, dans ce cas, ces fils élastiques conducteurs sont alors visibles au niveau d'au moins une face du textile et peuvent donc être endommagés, notamment lors du port du vêtement par l'utilisateur ou encore lors de son lavage en machine.

On connaît également, et tel que décrit dans le document EP 2 505 090, des vêtements instrumentés élastiques dans lesquels un fil conducteur élastique est positionné à la surface du textile, puis est recouvert d'un film de protection adhésif.

Cependant, un tel film de protection augmente la rigidité du vêtement instrumenté qui perd ainsi les caractéristiques intrinsèques de souplesse propre à un textile. Il en résulte un inconfort pour le porteur d'un tel type de vêtement. De plus, le matériau adhésif peut se dégrader dans le temps, notamment à cause de l'usure mécanique lié à l'usage des vêtements et/ou des lavages en machines successifs. Un vêtement instrumenté selon le préambule de la revendication 1 est connu de US 2006/0124193 A1. Un premier objectif est de permettre une protection efficace d'un fil conducteur élastique contre des agressions mécaniques extérieures, et notamment celles causées par les frottements et/ou le lavage en machine.

Un autre objectif de l'invention est de conserver la souplesse et l'élasticité du textile équipé d'un tel fil conducteur élastique.

### Exposé de l'invention

L'invention concerne donc un vêtement instrumenté comportant un textile tissé élastique comprenant :
- des fils élastiques non conducteurs agencés à la fois en sens chaine et en sens trame, et ;
- au moins un fil conducteur élastique destiné à former un bus pour transmettre au moins un signal électrique issu d'un capteur sensible à une caractéristique physiologique et/ou actimétrique du porteur du vêtement, le au moins un fil conducteur élastique étant positionné uniquement dans le sens trame du textile ;

Selon l'invention, un tel vêtement se caractérise en ce que le fil conducteur élastique est agencé dans un fourreau de protection, un tel fourreau étant formé par un tissage sélectif des fils élastiques non conducteurs agencés en sens chaine pour former deux nappes textiles parallèles superposées et localement disjointes.

Autrement dit, le ou les fils conducteurs élastiques sont invisibles et protégés par l'enveloppe que forme les deux nappes textiles parallèles. Pour réaliser un tel fourreau, on divise les fils de chaines élastiques non conducteurs en deux nappes et on les fait travailler alternativement suivant deux tissages agencés en parallèles.

Pour réaliser la nappe supérieure, on fait travailler uniquement les fils de chaine de la première nappe textile, c'est-à-dire que dans ce premier groupe de fils les fils de chaîne se séparent de manière régulière entre la position haute et la position basse, puis à chaque changement, un fil de trame est introduit. L'ensemble des fils de chaîne de la nappe inférieure restent quant à eux en position basse.

De même, pour réaliser la nappe inférieure, on fait travailler uniquement les fils de chaine de la deuxième nappe textile, c'est-à-dire que dans ce deuxième groupe de fils les fils de chaîne se séparent de manière régulière entre la position haute et la position basse, puis à chaque changement, un fil de trame est introduit. L'ensemble des fils de chaîne de la nappe supérieure restent quant à eux en position haute.

Au cours de la formation du fourreau, on introduit en trame un ou plusieurs fils conducteurs élastiques qui va être positionné entre les deux nappes parallèles. Pour cela l'ensemble des fils de chaine de la nappe supérieure se placent en position haute tandis que l'ensemble des fils de la nappe inférieure se placent en position basse. Le fil conducteur élastique est donc libre de se déplacer à l'intérieur d'un fourreau ce qui permet de conserver une grande souplesse au textile ainsi équipé.

On finalise le fourreau comme décrit précédemment, puis on le referme ensuite en faisant travailler ensemble les fils de chaine des deux nappes.

Avantageusement, le fil conducteur élastique peut être lié avec des fils non conducteurs de liage au moins au niveau des lisières du textile tissé, les fils de liage permettant de maintenir tendu le fil conducteur élastique.

Ainsi, le fil conducteur élastique est maintenu en tension à l'intérieur du fourreau, ce qui permet de faciliter les opérations ultérieures, de connexion avec un capteur notamment.

En pratique, les fils élastiques non conducteurs formant le fourreau peuvent posséder un coefficient d'élongation avant rupture inférieur à celui du fil conducteur élastique.

En d'autres termes, les fils élastiques non conducteurs formant le fourreau de protection sont moins élastiques que le fil conducteur élastique. Un tel agencement permet en outre de conférer une protection supplémentaire au fil conducteur élastique vis-à-vis de certaines sollicitations en traction.

Dans ce cas, la différence entre le coefficient d'élongation avant rupture du fil conducteur élastique et le coefficient d'élongation avant rupture des fils élastiques non conducteurs est comprise entre 5 et 50%. De façon préférentielle, cette différence peut également être comprise entre 10 et 30%.

A titre d'exemple, les fils élastiques non conducteurs peuvent avoir un coefficient d'élongation avant rupture d'environ 150% tandis que le fil conducteur élastique peut avoir un coefficient d'élongation avant rupture d'environ 190%.

Selon un mode de réalisation particulier, le fourreau peut être discontinu laissant apparaitre localement le fil conducteur élastique sur au moins une surface extérieure du vêtement instrumenté, cette discontinuité du fourreau formant une zone de connexion pour le fil conducteur élastique.

Autrement dit, une telle zone de connexion laisse apparaître ponctuellement le fil conducteur élastique à la surface du textile et permet de réaliser une connexion électrique entre fil conducteur élastique et un organe annexe tel un capteur notamment. La longueur visible de ce fil conducteur élastique peut avantageusement être comprise en 1 et 20mm. De cette façon, le fil conducteur élastique peut présenter une longueur libre suffisante permettant de le souder ou de le coller avec l'organe annexe.

Avantageusement, le vêtement peut comporter au moins un capteur sensible à une caractéristique physiologique et/ou actimétrique du porteur du vêtement, un tel capteur étant connecté au fil conducteur élastique au niveau de la zone de connexion.

Ainsi, le capteur comporte au moins une patte de connexion destinée à être solidarisée avec le fil conducteur élastique au niveau de la zone connexion. Une fois solidarisés entre eux, la connexion est alors recouverte par une membrane étanche qui peut notamment être formée par un matériau à base de silicone.

### Description sommaire des figures

La manière de réaliser l'invention, ainsi que les avantages qui en découlent, ressortiront bien de la description du mode de réalisation qui suit, donné à titre indicatif et non limitatif, à l'appui des figures dans lesquelles :
La figure 1 est une vue schématique de face d'un vêtement instrumenté, conforme à l'invention.
La figure 2 est une vue schématique en coupe transversale du textile élastique, conformément à l'invention.
Les figures 3 et 4 sont des vues schématiques de face représentant des détails de différentes zones d'un textile tissé, conformément à l'invention.

### Manière de décrire l'invention

Comme déjà évoqué, l'invention concerne un vêtement instrumenté comportant un textile tissé élastique.

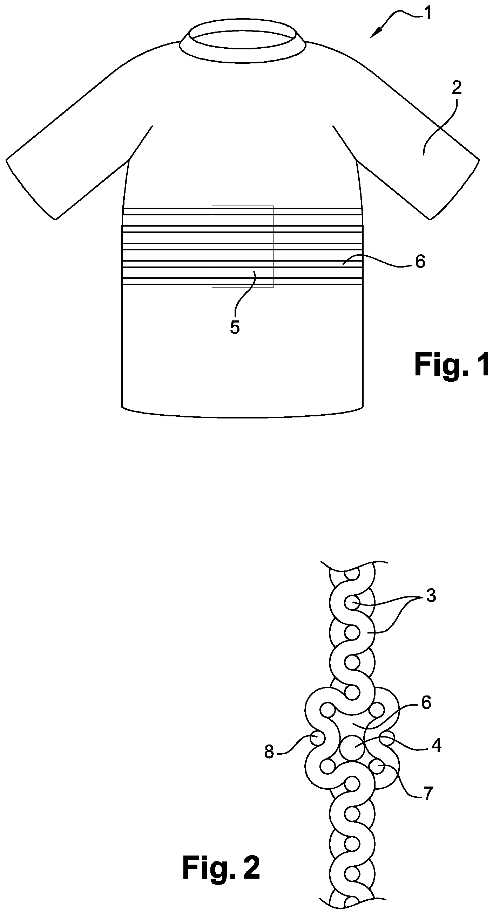

Tel que représenté à la figure 1, le vêtement 1 comporte un textile tissé élastique 2 permettant à un utilisateur de pratiquer notamment une discipline sportive ou toute activité physique.

Un tel vêtement 1 comporte ainsi un capteur 5, sensible à une caractéristique physiologique et/ou actimétrique du porteur du vêtement. En outre, un tel capteur **5** est connecté électriquement avec des fils conducteurs formant un bus de connexion permettant de transmettre différents signaux électriques entre le capteur **5** et une unité de traitement non représentée. Le capteur **5** est avantageusement choisi parmi le groupe comportant les capteurs permettant de mesurer la fréquence et la variabilité cardiaque (électrodes ECG), la fréquence respiratoire, la température cutanée, le flux thermique, le flux hydrique, l'oxygénation sanguine, l'oxygénation musculaire, la composition chimique de la sueur, la bio-impédance, la position, la vitesse ou l'accélération de tout ou partie du corps du porteur du vêtement.

Un tel vêtement **1** est ainsi formé par le tissage de fil élastique non conducteur agencé à la fois en sens chaîne et trame, et permettant de réaliser au moins un fourreau de protection **6** à l'intérieur duquel un fil conducteur élastique peut-être positionné.

En effet, tel que représenté à la figure 2, des fils élastiques non conducteurs **3** agencés à la fois en chaîne et trame permettent de définir deux nappes textiles **7, 8** parallèles et localement disjointes pour former le fourreau de protection **6.**

Un fil conducteur élastique **4** est maintenu à l'intérieur de ce fourreau de protection **6** de façon à protéger le fil conducteur élastique **4,** des agressions extérieures et notamment les frottements dus au lavage en machine.

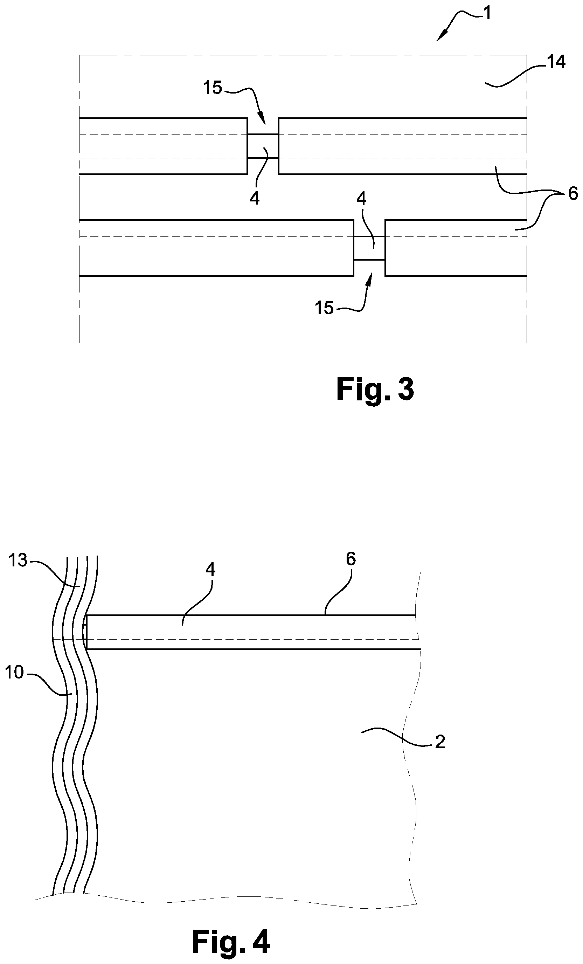

Tel que représenté à la figure 3, de tels fourreaux de protection **6** peuvent être localement discontinus de façon à laisser apparent une portion du fil conducteur élastique **4.** Une zone de connexion **15** pour le fil conducteur élastique **4** est alors visible au niveau de la face extérieure **14** du vêtement **1.** De telles interruptions peuvent notamment présenter une longueur d'environ 1 à 20 millimètres et préférentiellement avoir une longueur comprise entre 4 et 10 millimètres. De bons résultats ont par ailleurs été obtenus avec des interruptions du fourreau de protection d'une longueur d'environ 6mm.

Tel que représenté à la figure 4, le fil conducteur élastique **4** peut être maintenu au niveau de son extrémité au moyen de fils élastiques non conducteurs de liage **13** positionnés au moins au niveau d'une lisière **10** du textile tissé élastique **2.**

Un tel liage des extrémités du fil conducteur élastique **4** permet alors de le maintenir tendu à l'intérieur du fourreau **6** et en facilite les opérations de connexion, notamment pour réaliser sa soudure ou son collage avec un capteur.

Il ressort de ce qui précède qu'un vêtement instrumenté conforme à l'invention présente de nombreux avantages, et notamment :
- il est entièrement réalisé avec des matériaux textiles et présente ainsi une souplesse importante garantissant au porteur du vêtement ;
- les fils conducteurs possèdent une liberté de mouvement à l'intérieur des fourreaux de protection ce qui a pour effet d'améliorer encore la souplesse du vêtement ;
- il peut être lavé sans craindre un endommagement des fils conducteurs protégés à l'intérieur des fourreaux ;
- il peut présenter n'importe quel type de design.

## Revendications

1. Vêtement instrumenté (1) comportant un textile tissé élastique (2) comprenant :
- des fils élastiques non conducteurs (3) agencés en sens chaine et ;
- au moins un fil conducteur élastique (4) destiné à former un bus pour transmettre au moins un signal électrique issu d'un capteur (5) sensible à une caractéristique physiologique et/ou actimétrique du porteur du vêtement (1), ledit au moins un fil conducteur élastique (4) est agencé dans un fourreau de protection (6), ledit vêtement (1) étant **caractérisé en ce que** le textile tissé comprend des fils élastiques non conducteurs aussi dans le sens trame, le au moins un fil conducteur élastique (4) étant positionné uniquement dans le sens trame du textile (2) et ledit fourreau (6) étant formé par un tissage sélectif des fils élastiques non conducteurs (3) agencés en sens chaine pour former deux nappes textiles (7, 8) parallèles superposées et localement disjointes.

2. Vêtement selon la revendication 1, **caractérisé en ce que** ledit fil conducteur élastique (4) est lié avec des fils non conducteurs de liage (13) au moins au niveau des lisières (10) du textile tissé (2), lesdits fils de liage (13) permettant de maintenir tendu le fil conducteur élastique (4).

3. Vêtement selon la revendication 1, **caractérisé en ce que** les fils élastiques non conducteurs (3) formant ledit fourreau (6) possèdent un coefficient d'élongation avant rupture inférieur au coefficient d'élongation avant rupture du fil conducteur élastique (4).

4. Vêtement selon la revendication 3, **caractérisé en ce que** la différence entre le coefficient d'élongation avant rupture dudit fil conducteur élastique (4) et le coefficient d'élongation avant rupture les fils élastiques non conducteurs (3) est comprise entre 5 et 50%.

5. Vêtement selon la revendication 1, **caractérisé en ce que** ledit fourreau (6) est discontinu laissant apparaitre localement ledit fil conducteur élastique (4) sur au moins une surface extérieure (14) dudit vêtement instrumenté (1), cette discontinuité du fourreau (6) formant une zone de connexion (15) pour le fil conducteur élastique (4).

6. Vêtement selon la revendication 5, **caractérisé en ce qu'**il comporte au moins un capteur (5) sensible à une caractéristique physiologique et/ou actimétrique du porteur du vêtement (1), ledit capteur (5) étant connecté au fil conducteur élastique (4) au niveau de ladite zone de connexion (15).

## Patentansprüche

1. Instrumentiertes Kleidungsstück (1) mit einem elastischen Gewebe (2), umfassend:
- elastische, nicht stromleitfähige Fäden (3), die in Richtung der Kette angeordnet sind, und;
- mindestens einen elastischen, stromleitfähige Faden (4), der dazu bestimmt ist, einen Bus zu bilden, um mindestens ein elektrisches, von einem auf eine physiologische und/oder aktigraphische Eigenschaft des Trägers des Kleidungsstücks (1) sensibel reagierenden Sensor (5) ausgesendetes Signal zu übermitteln, wobei der mindestens eine elastische, stromleitfähige Faden (4) in einer Schutzhülle (6) angeordnet ist,
wobei besagtes Kleidungsstück (1) **dadurch gekennzeichnet ist, dass** das Gewebe elastische, nicht stromleitfähige Fäden auch in der Schussrichtung umfasst, wo der mindestens eine stromleitfähige elastische (4) Faden nur in Schussrichtung des Gewebes (2) positioniert ist und wo die Schutzhülle (6) aus einem selektiven Gewebe an elastischen, nicht stromleitfähigen und in Schussrichtung angeordneten Fäden (3) gebildet wird, um zwei parallele, übereinanderliegende und lokal getrennte Gewebelagen (7, 8) zu bilden.

2. Kleidungsstück gemäß Anspruch 1, **dadurch gekennzeichnet, dass** der stromleitfähige elastische Faden (4) zumindest auf Ebene der Stoffkanten (10) des Gewebes (2) mit nicht stromleitfähigen Verbindungsfäden (13) verbunden ist, wobei die Verbindungsfäden (13) ermöglichen, den stromleitfähigen elastischen Faden (4) gespannt zu halten.

3. Kleidungsstück gemäß Anspruch 1, **dadurch gekennzeichnet, dass** die elastischen, nicht stromleitfähigen Fäden (3) die Hülle (6) bilden und einen Bruch-Dehnungskoeffizienten besitzen, der geringer ist als der Bruch-Dehnungskoeffizient des stromleitfähigen elastischen Fadens (4).

4. Kleidungsstück gemäß Anspruch 3, **dadurch gekennzeichnet, dass** der Unterschied zwischen dem Bruch-Dehnungskoeffizienten des stromleitfähigen elastischen Faden (4) und dem Bruch-Dehnungskoeffizienten der elastischen, nicht stromleitfähigen Fäden (3) zwischen 5 und 50% beträgt.

5. Kleidungsstück gemäß Anspruch 1, **dadurch gekennzeichnet, dass** die Hülle (6) unterbrochen ist, und lokal den stromleitfähigen elastischen Faden (4) auf mindestens einer Außenfläche (14) des instrumentierten Kleidungsstücks (1) erscheinen lässt, wobei diese Diskontinuität der Hülle (6) einen Anschlussbereich (15) für den stromleitfähigen elastischen Faden (4) bildet.

6. Kleidungsstück gemäß Anspruch 5, **dadurch gekennzeichnet, dass** es mindestens einen Sensor (5) umfasst, der auf eine physiologische und/oder aktigraphische Eigenschaft des Trägers des Kleidungsstücks (1) sensibel reagiert, wobei der Sensor (5) an den stromleitfähigen elastischen Faden (4) im Bereich des Anschlussbereichs (15) angeschlossen ist.

## Claims

1. Instrumented garment (1) comprising an elastic woven fabric (2) comprising:
• nonconductive elastic yarns (3) arranged in the warp direction and;
• at least one elastic conductive yarn (4) designed to form a bus for the transmission of at least one electrical signal from a sensor (5) sensitive to a physiological and/or actimetric characteristic of the wearer of the garment (1) the said at least one conductive yarn (4) being arranged in a protective sleeve (6),
the said garment (1) being **characterized in that** the woven textile comprises nonconductive elastic yarns in the weft direction with the at least one elastic conductive yarn (4) being placed solely in the weft direction of the textile (2) and the said sleeve (6) being formed by the selective weaving of nonconductive elastic yarns (3) arranged in the warp direction to form two parallel overlaid and locally separated textile layers (7, 8).

2. Garment according to claim 1, **characterized in that** the said conductive elastic yarn (4) is linked with the nonconductive binding yarns (13) at least at the edges (10) of the woven textile (2), the said binding yarns (13) being used for holding the conductive elastic yarn (4) taut.

3. Garment according to claim 1, **characterized in that** the nonconductive elastic yarns (3) forming the said sleeve (6) have an elongation at rupture coefficient less than the elongation at rupture coefficient of the conductive elastic yarn (4).

4. Garment according to claim 3, **characterized in that** the difference between the elongation at rupture coefficient of the said conductive elastic yarn (4) and the elongation at rupture coefficient of the nonconductive elastic yarns (3) is included between 5 and 50%.

5. Garment according to claim 1, **characterized in that** the said sleeve (6) is discontinuous, leaving the said conductive elastic yarn (4) visible on at least one external surface (14) of the said instrumented garment (1), wherein this discontinuity in the sleeve (6) forms a connection zone (15) for the conductive elastic yarn (4).

6. Garment according to claim 5, **characterized in that** it includes at least one sensor (5) sensitive to a physiological and/or actimetric characteristic of the wearer of the garment (1), the said sensor (5) being connected to the conductive elastic yarn (4) in the said connection zone (15).
